# EUROPEAN PATENT APPLICATION

(11) **EP 0 979 871 A1**
(43) Date of publication of application: **16.02.2000**
(21) Application number: 98912716.2
(22) Date of filing: 08.04.1998
(51) Int. Cl.: C12N 15/12

(54) **NOVEL dlg FAMILY MOLECULE, POLYNUCLEOTIDE ENCODING THE SAME, ANTIBODY AGAINST THE SAME, AND METHOD FOR DETECTING dlg GENE**

(30) Priority: 14.04.1997 JP 11184697
(71) Applicant: Sumitomo Electric Industries, Ltd., Osaka-shi, Osaka 541-0041 (JP)
(72) Inventor: NAKATA, Motomi, Sumitomo Elect. Industries Ltd, Yokohama-shi, Kanagawa 244-8588 (JP); NAKAMURA, Hideo, Kumamoto-shi, Kumamoto 862-0962 (JP); SAYA, Hideyuki, Kumamoto-shi, Kumamoto 862-0970 (JP)
(74) Representative: Baldock, Sharon Claire
(86) International application number: JP9801611
(87) International publication number: WO9846745

(57) **Abstract**

This invention provides a novel dlg family molecule derived from human being, an antibody against the same and polynucleotides encoding the molecule. The present invention also provides a polynucleotide probe for detecting the polynucleotide in a specimen. This novel dlg family molecule and the polynucleotide encoding the molecule are significant in the clarification of cancer repression effect and the development of carcinostatic substance.

## Description

### Technical Field

This invention relates to a novel dlg family molecule, polynucleotide encoding the molecule, antibodies against the molecule and the polynucleotide in specimens.

### Background Art

Up to the present time, several cancer-inhibiting genes have been isolated in *drosophila* and genetic analysis of the genes has been made. Mutation of these genes in the germ line serves as recessive lethal. Among these genes, that (referred to as dlg gene hereinafter) generally called a dlg (discs large 1) is isolated, and the deletion thereof is known as the cause of excessive proliferation of the new born of imaginal discs. (Wood et al., Cell, Vol. 66, p 451-464, 1991.) In addition, it has been known that the dlg gene possesses three copies of the DHR domain (discs-large homologous region), a SH3 motif, and a domain homologous to the guanylate kinase of yeast.

Recently, several proteins that are homologous to dlg (or dlg molecule) that is a protein encoding the dlg gene have been identified. These proteins are considered to be a new protein family referred to as "MAGUK (membrane-associated guanylate kinase homology)." (Wood et al., Mechanism of Development, 44, p 85-89, 1993.) Hereinafter, this protein family is referred to as "dlg family." A protein belonging to this family is referred to as "a dlg family molecule." Further, if the animal species from which the protein is derived is designated, the species name is prefixed to "dlg family molecule" to express it. For example, when the molecule is derived from human being, it is expressed as "human dlg family molecule (or hdlg family molecule)."

These dlg family molecules, which have been known, include rat SAP-97, rat PSD-95, rat SAP-90 derived from rats; hdlg1, hdlg2, ZO-1, ZO-2, and human p55 derived from human being. Further, the genes encoding these dlg family molecules have been known. The above-mentioned human dlg1 (hdlg1) molecule has been known as binding to Protein 4.1 and existing on a cell membrane. In addition, the hdlg1 gene encoding the dlg1 has been known as a gene encoding an about 100 kd protein and is expressed in lymphocytes, heart, brain, spleen, lung, liver, muscle, and kidney among human tissues. (Leu et al., Proc. Natl. Acad. Sci. USA, Vol. 91, p 9818-9822, 1994)

### Disclosure of Invention

Since the dlg molecule of flies has the inhibitory effect on excessive proliferation of imaginal discs, the present inventors presumed that the dlg family molecules in vertebrate, particularly in mammal have the inhibitory effect on the excessive proliferation of cells, namely the effect of a kind of cancer-inhibiting factors, and therefore considered it would be significant to find out novel dlg family molecules in human being for the clarification of the cancer-inhibiting effect, the development of carcinostatic substances, etc. attained by using the novel dlg family molecules or the genes thereof.

Due to this significance, an object of the present invention is to provide novel human dlg family molecules and polynucleotide (DNA or RNA) encoding the novel human dlg family molecuels.

Another object of the present invention is to provide antibodies against the novel human dlg family molecules and useful in clarifying the function of the dlg family molecules.

Moreover, a further object of the present invention is to provide polynucleotide probes for detecting the genes of the novel dlg family genes in specimens.

As a result of extensive researches for attaining the above-mentioned objects, the present inventors have successfully discovered the genes encoding the novel dlg family molecules from cDNA library of human prostate. This invention has thus been accomplished. The present inventors have also succeeded in isolating and identifying the dlg family molecules encoded by the genes encoding the novel dlg family molecules. The novel dlg family molecules obtained by the present invention will be referred to as P-dlg hereinafter, and the genes encoding the P-dlg as P-dlg genes.

### Brief Description of Drawings

Fig. 1 is a photograph of electrophoresis showing the results of northern blotting where the cDNA fragments of P-dlg are hybridized to mRNAs of various human tissues.
Fig. 2 is a photograph of electrophoresis showing the results of northern blotting where the cDNA fragments of P-dlg are hybridized to mRNAs of various human tissues.
Fig. 3 is a photograph of electrophoresis showing the results of northern blotting where the cDNA fragments of P-dlg are hybridized to mRNAs of various human tissues.
Fig. 4 is a photograph of electrophoresis showing the results of northern blotting where the cDNA fragments of P-dlg are hybridized to mRNAs of various human cell strains.
Fig. 5 is a graph showing the results of ELISA measurement of the titers of an antibody against the P-dlg peptide.

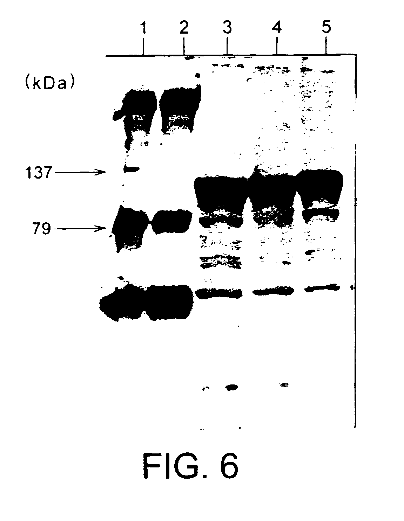

Fig. 6 is a photograph of electrophoresis showing the results of western blotting where the anti-P-dlg antibody was used against the extract of COS cells into which P-dlg was introduced. Here, Lane 1 and Lane 2 are the results of markers (molecular weight marker available from Bio-Rad Inc.); Lane 3, Lane 4 and Lane 5 represent the results of loading of the culture supernatants obtained by centrifugation of cell lysate of COS cells into which the P-dlg genes wer introduced. Lane, 3, Lane 4, Lane 5 represent the results of loading the culture supernatants of clone d, clone e and clone f, respectively.
Fig. 7 is a microscopic photograph showing the results of staining a PC-3 cell with an anti-P-dlg antibody.
Fig. 8 is graph showing the relationship among fragments A to C during the process of obtaining P-dlg of the present invention.

### Best Mode for Carrying Out the Invention

The embodiments of the present invention will be explained in detail hereinafter. Here, where the gene represents DNA obtained by reverse transcription of mRNA existing in nature (including DNA obtained by amplifying said DNA), it is referred to as "cDNA" to clarify the meaning.

### (P-dlg molecules and Polynucleotide Encoding the Same)

P-dlg molecules and polynucleotide encoding the same of the present invention can be obtained according to the process that will be described in details hereinafter.

Specifically, (1) On the basis of the information of the gene encoding the dlg family molecules of *drosophilia*, a base sequence having homology to the gene is obtained by homology search using EST database. (2) On the basis of thus obtained information of the base sequence, a primer for 5' elongation is synthesized. Then, a cDNA fragment is obtained by means of PCR using a cDNA library from human fetal brain as a template. (3) Further, on the basis of the base sequence information of the obtained fragment, a base sequence having homology to the fragment is obtained by homology search using EST database. (4) Subsequently, these base sequences are joined with each other, and on the basis of the base sequence information at the termination part of thus joined base sequence, a primer for amplifying the DNA fragment comprising this base sequence is synthesized. (5) This primer is used to produce an amplified product of a fragment having said primer at its terminus by means of PCR using the cDNA library from a human fetal as a template. (6) Then, a tissue wherein said amplified product is intensively expressed is identified, and elongation reaction of the DNA fragment toward 5'-side is conducted by means of PCR using cDNA library of the identified tissue as a template. For the elongation reaction, a primer for 5'-elongation synthesized on the basis of the base sequence information of said amplified product is used. (7) The above-described operations are repeated as occasion demands until the base sequence information of a full-length cDNA is obtained. (8) Furthermore, according to the obtained base sequence information of cDNA, a full-length cDNA (i.e., P-dlg cDNA) is obtained by means of PCR. There are no particular limitations to the method for determination of the base sequences. For example, the Taq cycle sequencing method as described in Biotechniques, Vol. 7, p 494-499, 1989 can preferably be used.

Further, on the basis of the information of the base sequence of the full-length cDNA obtained by the above-described process, it becomes possible to determine the amino acid sequence of P-dlg of the present invention encoded by this cDNA.

Furthermore, as will be explained hereinbelow, the northern blotting using the fragment of the P-dlg cDNA obtained as described above makes it possible to find out the tissue (for example, prostate, placenta, thyroid gland, spinal cord, trachea and adrenal gland) wherein P-dlg genes are expressed.

Furthermore, as will be explained hereinafter, antibodies (referred to as anti-P-dlg antibodies hereinafter) against the P-dlg obtained by above-mentioned operations or oligopeptides having a partial amino acid sequence of the P-dlg can be obtained. These antibodies can be used in function clarification of P-dlg expression, the clarification of signal transmission system and the like.

Further, as will be explained hereinafter, on the basis of the base sequence of P-dlg genes obtained as described above, the antisense polynucleotides can be used in experiments for the researches and development in relation to P-dlg genes, or in diagnostic agent for diagnosing the diseases in relation to P-dlg genes.

The novel human dlg family molecules according to the present invention and determined as described above have an amino acid sequence set forth in SEQ ID NO: 1 of the Sequence Listing, but not limited thereto. These molecules include molecules wherein one or more amino acids of the above amino acid sequence are substituted, deleted or added, and the molecules have a function of reacting with an antibody against Lys-Glu-Gln-Arg-Asp-Pro-Ile-Tyr-Leu-Arg-Asp-Lys-Val-Thr-Gln-Arg-His-Ser-Lys-Glu.

There are no particular limitations to the method for comparing the amino acid sequences. For example, the method is feasible through commercially available programs such as GENETYX (registered trademark)- CD program Ver. 34 (available from Software Development Inc.).

An example of genes encoding the novel human dlg family molecules according to the present invention is polynucleotide consisting of a base sequence set forth in SEQ ID NO:2 of the sequence listing, but the present invention is not limited to this base sequence. Natural or artificial variations make it possible to alter part of the structure of a polynucleotide without changing the activity of a polypeptide (which is its principal function) encoded by the polynucleotide. See, for example, "Molecular Cloning 2nd Edition," Cold Spring Harbor Laboratory Press, p 15.1-15.113, 1989. That is, in the case of P-dlg according to this invention, it is also possible to make substitution, deletion or addition of one or more amino acids in the amino acid sequence set forth in SEQ ID NO:1 of the Sequence Listing, without changing the principal function of the polypeptide. These are referred to as "P-dlg variants" in this invention. Here, the polypeptide has the above-mentioned function, that is, a function to react with the antibodies against Lys-Glu-Gln-Arg-Asp-Pro-Ile-Tyr-Leu-Arg-Asp-Lys-Val-Thr-Gln-Arg-His-Ser-Lys-Glu, as the principal function.

Further, because of degeneracy in genetic codes, it is possible to substitute at least part of bases in the base sequence of the polynucleotide with other kinds of bases without altering the amino acid sequence of a polypeptide produced by the polynucleotide. Therefore, the polynucleotides of this invention that encode the P-dlg and P-dlg variants include all those which contain any patterns of degeneracy capable of encoding said P-dlg or P-dlg variants.

### (Probes for Screening P-dlg Genes)

A probe to screen cDNA libraries for the P-dlg genes in the specimens can be selected from a DNA having 12 or more consecutive bases of the base sequence set forth in SEQ ID NO:2 of the Sequence Listing or a polynucleotide that hybridizes to said DNA. The sequences having the greater number of bases than that described above are recognized as the sequences having specificity. Here, those with GC contents of from 30 to 70% are preferably usable. Particularly preferable are DNAs having 15 bases or more and polynucleotides that hybridize to said DNAs. The polynucleotides that are used as probes may be derivatives modified by various substituents. With respect to cDNA libraries that are used for screening with the aid of said probes, those prepared from human mRNAs can preferably be used. A group of cDNAs selected by random sampling from these cDNA libraries may be employed as samples for screening. Commercially available cDNAs are also usable.

### (Acquisition of P-dlg and P-dlg variants)

There are no particular limitations to the method for acquiring P-dlg and P-dlg variants, and various kinds of publicaly known methods on the basis of P-dlg gene information can be employed. For example, they can be acquired by genetic engineering technique or chemical synthesis. According to the genetic engineering technique, a plasmid into which the gene is introduced is prepared on the basis of the information of the gene encoding P-dlg or P-dlg variants. Further, according to methods known in the art, it is possible to introduce the plasmid into a suitable host such as *E. coli*, to obtain a transformant expressing a protein. For example, it is feasible by the method as described in "Cell Engineering Protocols," Shujun-sha Co. Ltd., p 104-106, 1991. P-dlg can be produced by culturing a transformant thus obtained, amplifying the gene, and thereby expressing protein. Subsequently, a product thus cultured is recovered, and through manipulations such as concentration, solubilization, dialysis, and various chromatographic techniques as occasion calls, the P-dlg and P-dlg variants according to this invention can be purified.

A variety of references are available for the cultivation of transformants, and for example, it is feasible through the method as described in "Microorganism Experimental Methods, p97-134," The Japanese Society of Biochemistry, Incorporated Foundation, ed. Tokyo Kagaku Dojin, 1992. According to methods known in the art, it is also possible to express P-dlg on the basis of the base sequences described in this invention. In this case, any of bacteria such as *E. coli*, yeast, and animal cells is usable as a host. Particularly, animal cells are preferable. For the introduction of genes into cells, the liposome method, the electroporation method, and the like can be used. Particularly, it is preferable to use the DEAE-dextran method (available from Pharmacia Inc.).

For purifying P-dlg from the resulting cultured products, there are methods, such as immunoprecipitation salting out, ultrafiltration, isoelectric precipitation, gel filtration, electrophoresis, ion-exchange chromatography, a variety of affinity chromatography such as hydrophobic chromatography and antibody chromatography, chromatofocusing, adsorption chromatography, and reverse phase chromatography, and it may be carried out by selecting any of them as appropriate.

Also, in the production stage P-dlg to be produced may be prepared by transformants as fusion peptides with other polypeptides. In this case, the purification step requires a manipulation that slices out the P-dlg by treatment with a chemical substance such as bromocyanogen or with an enzyme such as protease.

Also, the protein can be obtained by using various oligopeptide synthesizing methods on the basis of the information of the amino acid sequence set forth in SEQ ID NO: 1 of the Sequence Listing.

### (Antisense Polynucleotides)

The present invention includes antisense polynucleotides having base sequences complementary to the genes encoding the dlg family molecules. Further, the present invention includes antisense polynucleotides and derivatives thereof corresponding to polynucleotides whose base sequence lengths are 15 or more bases, among the aforementioned polynucleotides. Also the present invention includes antisense polynucleotides and derivatives thereof corresponding to polynucleotides whose base sequence lengths are from 15 to 30 bases, among the aforementioned polynucleotides. The P-dlg antisense polynucleotides of this invention embrace all of those in which a plurality of nucleotides comprising bases, phosphoric acids, and sugars are bonded, including those artificially synthesized but not present in nature; their representatives are DNAs and mRNAs.

Also, the polynucleotides and antisense polynucleotide derivatives of this invention embrace all those analogous to polynucleotides in terms of their stereochemical structures or functions. For example, they may be polynucleotides 3'-or 5'-termini of which are bound to other substances, or may be those having undergone modifications such as substitution, deletion, and addition in at least parts of any of bases, sugars, and phosphoric acids of the polynucleotides, or those having bases, sugars or phosphoric acids not present in nature, or those having skeletons other than sugar-phosphoric acid skeletons.

The antisense polynucleotides or derivatives thereof according to the present invention may be ones that hybridize to any part of the polynucleotides encoding P-dlg or P-dlg variants. It is preferable that they comprise base sequences complementary to part of mRNA encoding the whole or part of P-dlg or a P-dlg variant and can hybridize to said mRNA.

Thus obtained antisense polynucleotides and derivatives thereof can effectively be used as polynucleotide probes for investigating the presence, as well as expression state, of polynucleotides encoding P-dlg or a P-dlg variant in tissues or cells. They can also be used as polynucleotide probes for diagnosing various diseases involving P-dlg or P-dlg variants.

Moreover, preferred as probes for the above mentioned purposes of use are ones that have 12 bases or more and GC contents of from 30 to 70%. In general, if the sequence is a base sequence containing 15 bases or more, it is considered to be specific (Protein, Nucleic Acid, and Enzyme, Vol. 38, p 754-765, 1994). Accordingly, particularly preferred are ones that have 15 bases or more and GC contents of from 30 to 70%.

Also, the antisense polynucleotides or derivatives thereof can be used to regulate the expression of P-dlg or P-dlg variants. It is expected that these hybridize to genes or mRNA encoding P-dlg or variants thereof and suppress the expression of P-dlg or variants thereof. Therefore, they can be used as therapeutics for functional disorders involving the P-dlg or P-dlg variants. In other words, antisense drugs can be developed based on said antisense polynucleotides or derivatives thereof.

The method to regulate the expression of a polypeptide by employing a polynucleotide containing the base sequence complementary to DNA or mRNA that encodes the polypeptide is called as "antisense method." The polynucleotide having such complementary base sequence is thought to regulate expression of the polypeptide at any of the following stages by binding to DNA or mRNA that carries genetic information and influencing the normal flow of transmission of the genetic information: (i) a transcription stage from a gene to pre-mRNA; (ii) a processing stage from the pre-mRNA to mature mRNA; (iii) a passing stage through nuclear membranes; and (iv) a translation stage to the protein.

It is preferable that the antisense polynucleotide or derivative thereof according to the present invention has a base sequence which is complementary to the P-dlg gene or mRNA corresponding to P-dlg and which comprises 12 or more, preferably 15 or more bases.

On the other hand, where a polynucleotide is to be incorporated into cells, it is unsuitable if its length is too long. The antisense polynucleotide or a derivative thereof according to this invention may be any length. However, in view of the fact that the antisense polynucleotide or a derivative according to this invention is to be incorporated into a cell and allowed to regulate the expression of P-dlg, it is preferable that the aforementioned antisense polynucleotide or derivative thereof has a base sequence which is complementary to the P-dlg gene or mRNA corresponding to P-dlg and which comprises 12 bases or more but 30 bases or less, more preferably 15 bases or more but 25 bases or less, and most preferably 18 bases or more but 22 bases or less.

With respect to the antisense polynucleotides and derivatives thereof according to this invention, a variety of derivatives can be obtained by using the antisense technology known in the art for the purpose of enhancing the effectiveness of the polynucleotides as pharmaceuticals. Namely, there can be obtained a variety of polynucleotide derivatives which are superior in binding strength with targeted DNAs or mRNAs, tissue selectivity, cell permeability, nuclease resistance, and intracellular stability.

In view of the ease of hybridization, it is considered to be advantageous that a polynucleotide or a derivative thereof are designed such that it has a base sequence complementary to the base sequence of a region which forms a stem loop of RNA (Clinical Immunology, Vol. 25, p 1200-1206, 1993). The antisense polynucleotide and the derivative thereof according to this invention can form a stem loop if necessary.

It is also expected that the antisense polynucleotides having sequences complementary to sequences existing in the vicinity of the translation start codon, or at a ribosomal binding site, the capping site or a splicing site are generally provided with a great inhibitory effect on expression. (Cancer and Chemotherapy, Vol. 20, No. 13, p 1899-1907.) Thus, a great expression inhibitory effect is attained in the polynucleotides or derivatives thereof according to this invention which contain sequences complementary to sequences existing in the vicinity of the translation start codon, or at a ribosomal binding site, the capping site, or a splicing site of a gene encoding P-dlg or P-dlg variants, or complementary to sequences existing in the vicinity of the translation start codon, or at a ribosomal binding site, the capping site, or a splicing site of mRNA corresponding to the genes.

At present, it is generally known that preferable derivatives are those which have an enhanced performance in at least one of nuclease resistance, tissue selectivity, cell permeability, and binding strength and that especially preferable derivatives are polynucleotide derivatives which have phosphorothioate bonds as their skeleton structures(Cancer and Chemotherapy, Vol. 20, No. 13, p 1899-1907, 1993). The polynucleotides or derivatives thereof according to this invention also embrace derivatives having these functions and structures.

For the method of producing the antisense polynucleotide derivatives according to this invention, a method as described in "Antisense Research and Applications," Michael J. Gait, p 290-299, CRC Press, Florida, 1993 can be used, for example.

If the antisense polynucleotides of this invention are DNAs or RNAs of the natural type, they can be obtained either by synthesis using a chemical synthesizer or by the PCR method that employs the gene encoding P-dlg or a P-dlg variant as a template. Also, among the derivatives, there are ones that can be synthesized using a chemical synthesizer (e.g., Type 394 available from Perkin Elmer Inc.), such as the methylphosphonate type and the phosphorothioate type. In this case, the synthesizing operation may be conducted according to the manual attached to the chemical synthesizer and the synthesized products as obtained can be purified by HPLC method using reverse phase chromatography or other methods to produce the desired antisense polynucleotides or derivatives thereof.

### (Anti-P-dlg Antibody)

The anti-P-dlg antibodies of this invention embrace either of polyclonal antibodies and monoclonal antibodies insofar as they are antibodies against P-dlg or the variants thereof according to the present invention.

They also embrace active fragments thereof and chimera antibodies containing the active fragments. In general, the antibody, i.e., immunoglobulin, has H and L chains and is classified into five classes (IgA, IgD, IgE, IgG, and IgM). Among these, IgA and IgG are further classified into subclasses with respect to the types of their H chains. The novel antibodies of this invention embrace all those belonging to these classes and subclasses.

Moreover, the antibody of this invention does not necessarily need to use its whole antibody molecule and can use a portion of the molecule (active fragment) insofar as it is active. As the active fragment, specifically named are F(ab')₂, Fab', Fab, Fv, recombinant Fv, and single-chain Fv. For example, F(ab')₂ and Fc' result if the antibody is digested with pepsin, and Fab and Fc result if digested with papain.

Although these active fragments can be used alone, they can be conjugated to substances such as albumin and polyethylene glycol, if necessary, and can be used as new conjugates. In many cases, such conjugates generally exhibit their effectiveness to the maximum degree without being decomposed for a long period of time in vivo. The method for attaching a substance such as albumin or polyethylene glycol to the active fragment is, for example, described in "Antibodies, A Laboratory Manual," Cold Spring Harbor Laboratory, p 77-81 and p 129-137, 1988. In general, employing divalent-reactive reagents such as SPDP (available from Amersham Pharmacia Biotech Inc.), SMPB (available from Pierce Inc.), and EMCS (Dotite Inc.), the active fragments can easily be conjugated to albumin or the like.

The anti-P-dlg antibodies of this invention can be obtained according to methods known in the art. For example, they can be obtained by referring to "Immunological Experimental Procedures," edited and published by The Immunological Society of Japan. For an immunogen, there may be used part of the P-dlg of this invention: namely, a polypeptide comprising eight or more consecutive amino acids out of the amino acid sequence set forth in SEQ ID NO:1 of the Sequence Listing. For example, there is a polypeptide comprising a specific amino acid sequence of KEQRDPIYLRDKVTQRHSKE, that is specific to P-dlg. Further, it is preferable to conjugate maleimidated KLH (hemocyanin from *Macroschisma sinensis*) to C which is appended to the C-terminus of the polypeptide. Also, the method of obtaining said P-dlg should not be questioned insofar as it has such purity as will allow its use for preparation of the antibodies.

Where the immunogen is a polypeptide comprising from 8 to about 20 amino acids, it may be conjugated to a carrier such as keyhole lympet hemocyanin (KHL) and used as an antigen.

Animals to be immunized with the immunogen may be any animals other human. Among animals that are used by those skilled in the art, animal species capable of producing the desired antibodies may preferably be selected for use.

The polyclonal antibodies may be obtained by purifying the resulting antisera. The purification can be a combination of the methods such as salting out, ion-exchange chromatography, and affinity chromatography.

The monoclonal antibodies may be obtained by ordinary methods for producing hybridomas: after fused cells are obtained, they are allowed to produce antibodies. For the cell fusion, techniques employing polyethylene glycol, Sendai virus, electric pulse, etc. are usable.

Other than the above, genetic engineering methods can also be used to obtain the monoclonal antibodies. For example, mRNA is extracted from the spleen cells or lymphocytes of an animal immunized with the P-dlg or part thereof according to this invention, or from the hybridoma producing the monoclonal antibody, and from the mRNA thus extracted a cDNA library is prepared. An antibody is then expressed by the cDNA library. The clone producing the antibody reactive to an antigen is obtained by screening the cDNA library, the resulting clone is cultured, and the desired antibody can be purified from the cultured mixture by the combination of methods such as salting out, ion-exchange chromatography, and affinity chromatography.

The antibodies of this invention can be used to detect the P-dlg of the invention, a variant thereof, or part of the foregoing existing in cells of prostate, placenta, thyroid gland, spinal cord, adrenal gland and trachea tissue. The antibodies can also be used to prepare antibody columns for use in purification of the P-dlg, a variant thereof, or part of the foregoing according to the invention, as well as to detect said P-dlg, a variant thereof, or part of the foregoing in respective fractions.

This invention will further be detailed with the illustration of examples below; however, the invention is not to be limited to these examples.

### (EXAMPLE 1) Identification of the P-dlg Molecule

### (1) Screening of Data Base using Internet [http://www.ncbi.nlm.nih.gov/dbEST/index.htlm]:

On the basis of the gene encoding the dig molecule of *drosophilia*, the inventors accessed the gene data base on Internet and homology search was conducted using a personal computer. Specifically, a fragment of EST referred to as H29224 is found when the inventors investigated EST existing in the 10th site of a chromosome from Unigene database (http://www.ncbi.nml.nih.gov/UniGene/index.html). This fragment was further studied to reveal that an EST fragement derived from the same clone was H29225. These two ESTs were searched with another batabase, i.e., TIGR database (http://www.tigr.org/tigr_home/tdb/) and the search result revealed that THC79238 having the following sequence was a fragment covering these two ESTs:

TCCAGGAGGC TCAGCATGTC TGAAGTCAAA GATGACAATA GCGCCACAAA GACGCTGTCA

GCGGCTGCAC GCCGGTSCTT TTTTCGGAGG AAACACAAGC ACAAACGCAG CGGGTCCAAG

GACGGGAAAG ACCTGCTCGC CTTGGATGCC TTTTCCAGTG ACTCCATTCC ACTCTTTGAA

GATTCGGTGA GCCTGGCCTA TCAGCGGGTC CAGAAGGTGG ACTGCACCGC TCTGAGGCCT

GTCCTGATTC TGGGGCCTTT GCTGGACGTG GTGAAGGAGA TGCTGGTGAA TGAGGCTCCT

GGCAAGTTCT GCAGATGTCC CCTTGAGGTG ATGAAGGCCT CCCAGCAGGC CATTGAGCGG

GGTGTCAAAG ATTGCCTGTT TGTCGACTAT AAGCGGAGAA GCGGCCATTT CGATGTGACC

ACTGTGGCGT CAATAWAGGA GATCACAGAA AAGAACCGAC ACTGCCTCCT GGACATTGCT

CCGCACGCTA TTGAGCGGCT CCACCACATG CACATCTACC CCATTGTCAT CTTCATCCAC
TACAAGAGCG CCAAGCACAT CAAGGAGCAG AGAGACCCCA TCTACCTGAG GGACAAGGTG
ACTCAGAGGC ATTCCAAAGA GCARTTTGAG GCGGCGCAGA AGCTTGAGCA GGAGTACAGC
AGGTACTTCA CAGGGGTCAT CCAGGGAGGA GCCCTGTCAA GCATTTGCAC TCAGATCTTG

The acquisition of novel human-dlg family molecules on the basis of this sequence was attempted.

### (2) 5'-Side Elongation Reaction

A termination codon was identified as a result of the detailed investigation in respect to the sequence of HTC79238 acquired on the basis of the foregoing database. Therefore, in order to acquire a full-length cDNA based on this sequence, elongation reaction toward the 5'-side where the start region for translation of genes was believed to exist was conducted by PCR operation.

### (I) Synthesis of Primers

Primer AS3 (5'-GCATCCAAGGCGAGCAGGTCTTT-3'), primer AS2 (5'-TGCAGCCGCTGACAGCGTCTTTGT-3') and primer T7 (5'-AATACGACTCACTATAG-3') were designed and synthesized with a DNA synthesizer (ABI model 392). The synthesized DNA primers were dissolved in distilled water to give a concentration of 20 pmol/µl. This was used as PCR primers to perform the PCR operation as described below.

### (II) PCR Operation

First, PCR operation employing AS3 primer toward only one side (referred to as single PCR operation in some cases hereinafter) was conducted.

The PCR operation was as follows: A solution having the composition (15 µl in total) of cDNA (1µg/µl) 2 µl from human fetal brain, a dNTP mixed solution (respective NTPs were dissolved in distilled water at concentrations of 20 pmol/µl) 1 µl, Primer AS3 (20 pmol/µl) 1 µl, 10-fold concentrated PCR buffer (400 mM Tris-HCl (pH 8.3), 1 M KCl, 100 mM MgCl₂, 100 mM DTT) 1.5 µl, distilled water 9 µl, and Taq polymerase (5 units/µl) 0.5 µl was placed into a test tube. Mineral oil (15 µl) was overlaid on the solution and it was allowed to stand at 94 °C for 5 min. Subsequently, the following cycle was repeated 50 times for reaction: at 60 °C for 30 sec, followed by at 72 °C for 1.5 min, and followed by at 94 °C for 30 sec. Then the reaction was allowed to proceed at 55°C for 2 min, finally at 72 °C for 10 min to carry out the elongation reaction of the fragment, completing the PCR operation.

Next, this PCR product was used as a template to perform the second PCR as follows: A solution having the composition (20 µl in total) of the aforementioned PCR product (1µg/1µl) 5 µl, a dNTP mixed solution (respective NTPs were dissolved in distilled water at concentrations of 20 pmol/µl)1 µl, Primer AS2 (20 pmol/µl) 1 µl, Primer T7 (20 pmol/µl) 1 µl, 10-fold concentrated PCR buffer (400 mM Tris-HCl (pH 8.3), 1 M KCl, 100 mM MgCl₂, 100 mM DTT))2 µl, distilled water 9.5 µl, and Taq polymerase (5 units/µl) 0.5µl was placed into a test tube. Mineral oil (20 µl) was overlaid on the solution and it was allowed to stand at 94 °C for 5 min. Subsequently, the following cycle was repeated 40 times for reaction: at 60 °C for 30 sec, followed by at 72 °C for 1 min, and followed by at 94 °C for 30 sec. Then, reaction was allowed to proceed at 55 °C for 2 min and finally at 72 °C for 10 min to carry out the elongation reaction of the fragment, completing the PCR operation.

### (III) Determination of DNA Base Sequence

Direct DNA sequencing was conducted on the DNA fragment of about 0.6 kbp (referred to as fragment A hereinafter) obtained through the above-mentioned 5'-side elongation reaction according to the Dye-terminator method using Primer T7. Specifically, the PCR product obtained as describe above was subjected to the mini gel electrophoresis (0.75% agarose gel) to cut a band of a fragment of P-dlg out of the gel. The PCR product was recovered by means of Gene Clean (available from BIO 101 Inc.), and the band was ascertained by means of the mini gel electrophoresis (0.75% agarose gel).

Plasmid DNA 1 µl was taken up and diluted in 99 µl of TE. The diluted solution was measured at A260 and the DNA concentration was computed (A260, 1.0= 50 µg/ml). The diluted solution was further diluted with TE buffer so that its DNA concentration reached 1 µg/µl. DNA sequencing was conducted according to the Dye-terminator method (ABI Model 373A).

### (3) Screening of Database

Based on the DNA sequence obtained in item (2), homology search was conducted using EST database (NCBI site, [http://www.ncbi.nlm.nih.gov/dbEST/index.htlm]). (EST database is a database where sequences obtained as a result of an acquisition of mRNA fragments and a determination of their cDNA sequences are registered.) As a consequently, a sequence (expressed as THC90513) corresponding to a portion of above-mentioned sequence was ascertained. Three sequences, i.e., THC90513, fragment A obtained as described above and THC79238 were integrated from 5'-side to 3'-side on the computer. Subsequently, the DNA sequence obtained by integrating these three sequences was further searched using EST database, and W26281 as a sequence that is in partial agreement with the foregoing sequence was ascertained.

### (4) 5'-side Elongation Reaction (II)

### (I) Elongation on the Computer

W26281 and the sequence obtained from the above-mentioned three sequences were integrated from 5'-side to 3'-side to obtain a sequence of a DNA fragment of about 2.4kbp. Thereafter, 5'-side elongation reaction from the DNA fragment obtained by the foregoing operation was conducted by means of PCR operation using a cDNA library of human fetal brain.

### (II) Acquisition of cDNA fragment

5'-terminal region of the DNA fragment of about 2.4kbp obtained by the above-mentioned operation of item (4)(I) corresponds to the fragment of W26281, whereas 3'-terminal region to the fragment of THC79238. A sense primer (5'-AAAGACAACCCCAGGATTCGGAAG-3') was designed for the portion corresponding to the fragment of W26281, whereas an antisense primer (5'-CGTGAACTCCTCAGGGCGGTACTG-3') was designed for the portion corresponding to the fragment of THC79238. Employing these primers, PCR operation was performed again using the cDNA library of human fetal brain as a template so as to obtain a cDNA fragment (referred to as fragment B hereinafter) of the portion corresponding to the sequence of about 2.4kbp obtained in foregoing item (4)(I).

### (i) Synthesis of Primers

The sense primer and the antisense primer were synthesized with a DNA synthesizer (ABI model 392). The synthesized DNA primers were dissolved in distilled water to give a concentration of 20 pmol/µl. This was used as PCR primers to perform the PCR operation as described below.

### (ii) PCR Operation

The PCR operation was as follows: A solution having the composition (20 µl in total) of cDNA (1µg/µl) 5 µl from human fetal brain, a dNTP mixed solution (respective NTPs were dissolved in distilled water at concentrations of 20 pmol/µl) 1 µl, sense primer (20 pmol/µl) 1 µl, antisense primer (20 pmol/µl) 1 µl, 10-fold concentrated PCR buffer (400 mM Tris-HCl (pH 8.3), 1 M KCl, 100 mM MgCl₂, 100 mM DTT) 2 µl, distilled water 9.5 µl, and Taq polymerase (5 units/µl) 0.5 µl was placed into a test tube. Mineral oil (20 µl) was overlaid on the solution and it was allowed to stand at 94 °C for 5 min. Subsequently, the following cycle was repeated 40 times for reaction: at 60 °C for 30 sec, followed by at 72 °C for 1 min, and followed by at 94 °C for 30 sec. Then the reaction was allowed to proceed at 55°C for 2 min, and finally at 72 °C for 10 min to carry out the PCR operation.

### (III) Mini Gel Electrophoresis

After the reaction as described above, the PCR product was subjected to a mini gel electrophresis(0.75% agarose gel) . As a result, a band of about 2.4 kbp was observed. This band was considered as a portion of the gene encoding the novel dlg family molecules.

### (5) Analysis by Northern Blotting

Investigation of Expression of the DNA fragmment obtained according to the above described operation in respective tissues was conducted as follows:

A kit (Human Multiple Tissue Northern Blot I, II and Human Cancer Cell Line Multiple Tissue Northern Blot) was purchased from Clontech Inc.: the kit comprised 2 µg of poly(A)+RNA (mRNA) from a variety of human tissues and poly(A)+RNA (mRNA) from a variety of human cell strains blotted in each membrane.

DNA of fragment B obtained in item (4) was hybridized to the membranes, as described below. First, the DNA fragment was labeled with ³²P-CTP using a Random Primed Labeling Kit (available from Takara Co. Ltd.). Next, the labeled cDNA was hybridized to the membranes under high stringency conditions by following the description in Molecular Cloning: A Laboratory Manual 2nd Edition, p 7.39-7.52.

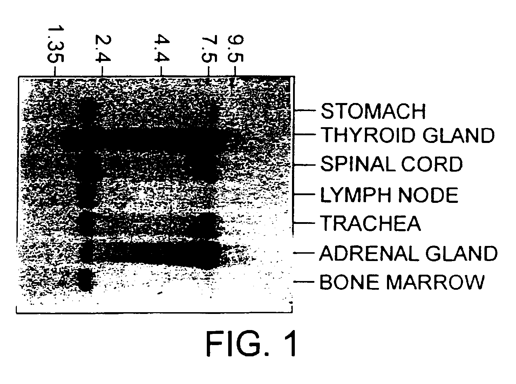

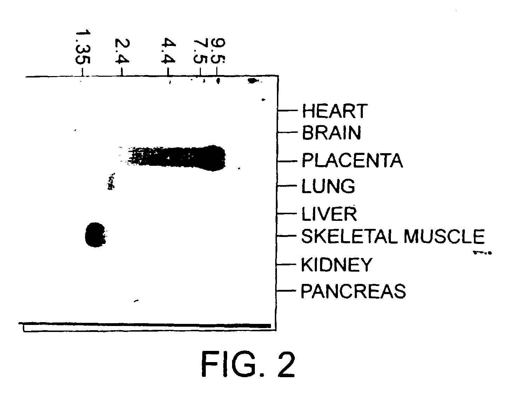

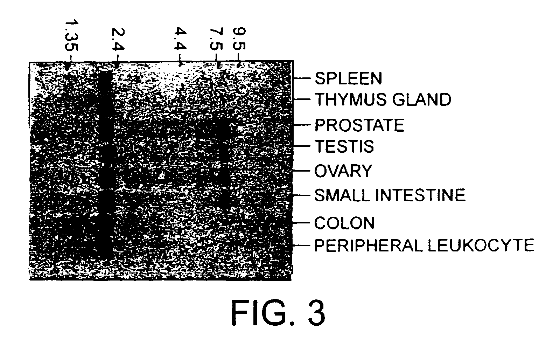

Photographs illustrating the results with regard to the respective tissues are shown in Figs. 1 to 3. It was learned from the photographs that P-dlg is expressed in prostate, placenta, thyroid gland, spinal cord, trachea and adrenal gland.

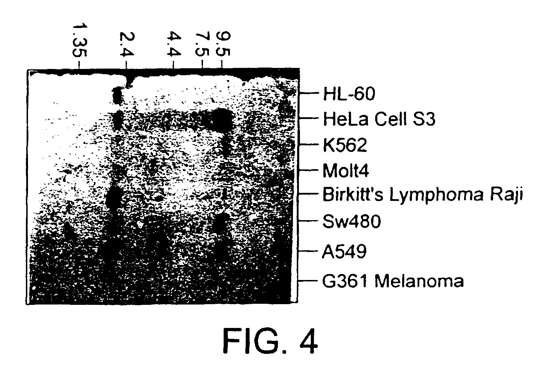

Fig. 4 is a photograph showing the results in respect to various cells. It is learned from this photograph that the P-dlg is expressed in Hela (human cervical carcinoma).

As mentioned above, the high expression was particularly found in prostate. Therefore, a cDNA library of human prostate was used as a template to attempt the acquisition of a full-length cDNA according to the operation that will be described hereinafter.

### (6) 5'-side Elongation Reaction (III)

### (I) Synthesis of Primers

Primer AS4 (5'-CGTGAACTCCTCAGGGCGGTACTG-3'), primer AS5 (5'-CTTCAGGCGGACGCCAGCCCT-3') and primer T3 (5'-ATTAACCCTCACTAAAG-3') were designed and synthesized with a DNA synthesizer (ABI model 392). The synthesized DNA primers were dissolved in distilled water to give a concentration of 20 pmol/µl. This was used as PCR primers to perform the PCR operation as described below. (II) PCR Operation (i)

Primer AS4 was employed to perform the single PCT operation.

The PCR operation was as follows: A solution having the composition (15 µl in total) of cDNA (1µg/µl) 2 µl from human prostate (purchased from Clontech Inc.), a dNTP mixed solution (respective NTPs were dissolved in distilled water at concentrations of 20 pmol/µl) 1 µl, Primer AS4 (20 pmol/µl) 1 µl, 10-fold concentrated PCR buffer (400 mM Tris-HCl (pH 8.3), 1 M KCl, 100 mM MgCl₂, 100 mM DTT) 1.5 µl, distilled water 9 µl, and Taq polymerase (5 units/µl) 0.5 µl was placed into a test tube. Mineral oil (15 µl) was overlaid on the solution and it was allowed to stand at 94 °C for 5 min. Subsequently, the following cycle was repeated 50 times for reaction: at 60 °C for 30 sec, followed by at 72 °C for 1.5 min, and followed by at 94 °C for 30 sec. Then the reaction was allowed to proceed at 55°C for 2 min, and finally at 72 °C for 10 min to carry out the elongation reaction of the fragment, completing the PCR operation.

### (III) PCR operation (ii)

Next, this PCR product was used as a template to perform the second PCR operation using primer AS5 (5'-CTTCAGGCGGACGCCAGCCCT-3') as the primer for 3'-portion and primer T3 (5'-ATTAACCCTCACTAAAG-3') for 5'-portion.

The second PCR operation was performed as follows: A solution having the composition (20 µl in total) of the aforementioned PCR product 5 µl, a dNTP mixed solution (respective NTPs were dissolved in distilled water at concentrations of 20 pmol/µl)1 µl, Primer AS5 (20 pmol/µl) 1 µl, Primer T3 (20 pmol/µl) 1 µl, 10-fold concentrated PCR buffer 2 µl, distilled water 9.5 µl, and Taq polymerase (5 units/µl) 0.5µl was placed into a test tube. Mineral oil (20 µl) was overlaid on the solution and it was allowed to stand at 94 °C for 5 min. Subsequently, the following cycle was repeated 40 times for reaction: at 60 °C for 30 sec, followed by at 72 °C for 1 min, and followed by at 94 °C for 30 sec. Then, reaction was allowed to proceed at 55 °C for 2 min and finally at 72 °C for 10 min to carry out the elongation reaction of the fragment, completing the PCR operation, thus obtaining a DNA fragment (referred to as fragment C hereinafter) of about 1.3kbp.

### (IV) Determination of DNA Sequence

Direct DNA sequencing on fragment C obtained from the above described operation was conducted according to the Dye-terminator method using Primer T3.

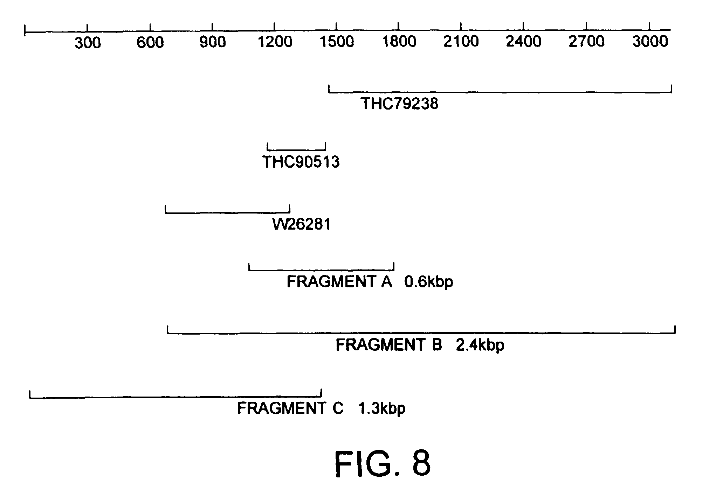

The sequences of three fragments, i.e., fragments A, B and C were thus determined. Fragment A was embraced in fragment B, and the sequence of a full-length gene was determined by fragments B and C. A relationship of the fragments is shown in Fig. 8.

### (7) Acquisition of a full-length cDNA

### (I) Synthesis of Primers

The sense primer (5'-TAGCAGACACTCTTGCCCTTGCA-3') was designed for 5'-terminal portion whereas the antisense primer (5'-TCACCTTCCCCCATTGTGGGAGGA-3') for 3'-terminal portion on the basis of the data of sequencing of the DNA fragment obtained from fragments B and C. Employing these primers, PCR operation was performed again using the cDNA library of human prostate as a template under the condition described below so as to obtain a full-length coding region.

The sense primer and the antisense primer were synthesized with a DNA synthesizer (ABI model 392). The synthesized DNA primers were dissolved in distilled water to give a concentration of 20 pmol/µl. This was used as PCR primers to perform the PCR operation as described below.

### (II) PCR Operation

The PCR operation was as follows: A solution having the composition (20 µl in total) of cDNA (1µg/µl) 5 µl from human prostate, a dNTP mixed solution (respective NTPs were dissolved in distilled water at concentrations of 20 pmol/µl) 1 µl, sense primer (20 pmol/µl) 1 µl, antisense primer (20 pmol/µl) 1 µl, 10-fold concentrated PCR buffer 2 µl, distilled water 9.5 µl, and Taq polymerase (5 units/µl) 0.5 µl was placed into a test tube. Mineral oil (20 µl) was overlaid on the solution and it was allowed to stand at 94 °C for 5 min. Subsequently, the following cycle was repeated 40 times for reaction: at 60 °C for 30 sec, followed by at 72 °C for 1 min, and followed by at 94 °C for 30 sec. Then the reaction was allowed to proceed at 55°C for 2 min, and finally at 72 °C for 10 min to carry out the PCR operation, thus obtaining a full-length cDNA.

### (III) Determination of Sequence

Sequencing of the full-length DNA was carried. As a result, a sequence containing a full length of a target gene was obtained as shown in SEQ ID NO: 2 of the Sequence Listing. This gene was named P-dlg gene. The amino acid sequence encoding P-dlg gene is shown in SEQ ID NO: 1 of the Sequence Listing

Furthermore, the P-dlg cDNA was incorporated into a PCGN vector (available from Invitorogen Inc.) and transformation was carried out by introducing said vector to *E. coli*(HB101). This transformant was deposited on February 14, 1997 in the National Institute of Bioscience and Human-Technology, Agency of Industrial Science and Technology, Ministry of International Trade and Industry locating at 1-1-3 Higashi, Tsukuba, Ibaragi, Japan and it was transferred on January 21, 1998 to International Deposition (Accession No. FERM BP-6234) under Budapest Treaty.

### (Example 2) Chromosomal Mapping

The chromosomal mapping of the full-length cDNA of P-dlg obtained in Example 1 was carried out. The analysis of the full-length cDNA of P-dlg using EST database revealed that a plurality of sequences registered in EST overlap a portion of the sequence of P-dlg cDNA. As a result, it was ascertained that P-dlg gene is located on chromosome 10 between WI7219 and WI4544 registered in EST database, specifically, 10q24 of the chromosome. (It is considered to be the same location where the aforementioned H29224 is mapped.)

### (EXAMPLE 3) Production of Antibodies against P-dlg

### (I) Preparation of Antigens

The peptide as described below, which is partial sequence of the P-dlg was synthesized with a peptide synthesizer (ABI Model 431A)

Sequence 1: A sequence which is peculiar to the P-dlg (sequence of 604th to 623rd amino acid): 21mer peptide produced by appending C(Cys) to the N-terminus of KEQRDPIYLRDKVTQRHSKE.

### (II) Preparation of Polyclonal Antibodies

Antibodies against the peptide prepared in item (I) was prepared by following the procedure as described below.
(i) The synthesized peptide (2 mg) was conjugated to 2 mg of maleimidated KLH (hemocyanin from *Macroschisma sinensis*, available from Pierce Inc.). The reaction was carried out according to the method as described in the kit from Pierce Inc.
(ii) 100 micrograms of this peptide-KLH was used for a single immunization. Namely, 100 µl of a peptide-KLH preparation (1 µg/ml), 0.5 ml of PBS and 0.5 ml of Freund complete adjuvant (available from Difco Inc.) were taken in a syringe and mixed to prepare an emulsion, which was used in the initial immunization.
(iii) This emulsion was subcutaneously inoculated to rabbits. The inoculation was separately done at four places on the back of a rabbit.
(iv) One week later, the second immunization was carried out. The adjuvant was changed to Freund incomplete adjuvant (available from Difco Inc.) from the second time to effect immunization. The other manipulations were the same as those in the first time. After the second time, immunization was done six times in total at intervals of one week.
(v) One week after the final immunization, blood was collected from the rabbits to obtain serum. After the collected blood was allowed to stand at room temperature for 3 h and sufficient coagulation was effected, centrifugation was done at 3,000 rpm for 5 min to recover the supernatant (serum).
(vi) This serum was salted out by adding 100% saturated ammonium sulfate to bring the final concentration to 50% saturation. This sample was centrifuged and fractions containing an antibody were precipitated. Subsequently, the precipitate was dissolved in PBS and was further dialyzed against PBS. Then, the antibody was purified by affinity chromatography using a Protein G Sepharose column (available from Amarsham Pharmacia Biotech Inc.). As a result, a total amount of 370 mg of an IgG fraction was obtained.
(vii) The aforementioned IgG fraction obtained through the manipulations was further purified by affinity chromatography using the peptide synthesized in item (i) and had been used in the immunization. The peptide column was prepared by binding the peptide synthesized in item (i) to NHS-activated Sepharose (available from Amersham Pharmacia Biotech Inc.) according to the Manual attached to the Sepharose. With the aid of this column, the purified IgG fraction as obtained in item (vi) was further purified by affinity chromatography. As a result, a total amount of 5 mg of a peptide-specific antibody was obtained.

### (III) Determination of Antibody Titers (ELISA)

Titers of the peptide-specific antibody obtained in item (II) were determined by ELISA.
(i) Antigen solution (the peptide of sequence 1) was dissolved in PBS to give a concentration of 25 µg/ml, and it was dispensed to each well of a 96-well ELISA plate (Xenobind, available from Xenopore Inc.), which was allowed to stand overnight at 4 °C.
(ii) The antigen solution was discarded and each 200 µl of Blockace (available from Dainippon Pharmaceuticals Co. Ltd.) diluted fourfold with distilled water was dispensed to each well. Blocking was carried out by allowing it to stand at room temperature for 2 h.
(iii) The blocking solution was discarded and the purified antibody to be examined as a primary antibody was added. A serially (in twofold) PBS-diluted solution of the purified antibody was added to each well at 50 µl/well so that its concentration could successively reach 10 µg/ml, 5 µg/ml, 2.5 µg/ml, etc. from the first row of the 96 wells. The concentration of the antibody in the final well proved to be about 0.004 µg/ml. In addition, IgG purified from the blood of a rabbit that was not immunized was used as a control. After being dispensed to each well, it was allowed to stand at room temperature for 1 h.
   Where the serum collected from a rabbit was used as a preparation containing the primary antibody, a 40-fold diluted solution using PBS was added to the first row of the 96 wells and a solution serially diluted (in twofold) with PBS was added successively.
(iv) The antibody preparation was discarded and the plate was washed with 0.05% Tween20/PBS four times. Next, each 50 µl of the biotinylated anti-rabbit IgG antibody (available from Vector Inc.) that had been 1000-fold diluted with PBS, which served as a secondary antibody preparation, was dispensed to each well, and it was allowed to stand at room temperature for 1 h.
(v) After the secondary antibody preparation was discarded, the plate was washed with 0.05% Tween20/PBS four times. 50 µl of an ABC solution diluted 1000-fold (available from Vector Inc.) was dispensed to each well, and it was allowed to stand at room temperature for 30 min.
(vi) Then, after the ABC solution was discarded, the plate was washed with 0.05% Tween20/PBS four times. Each 100 µl of OPD (orthophenylenediamine)-H₂0₂/PCB was dispensed to each well. After the reaction was quenched with 2N sulfuric acid upon sufficient coloring, the absorbance at 490 nm was measured with a microplate reader (available from Bio-rad Inc.).

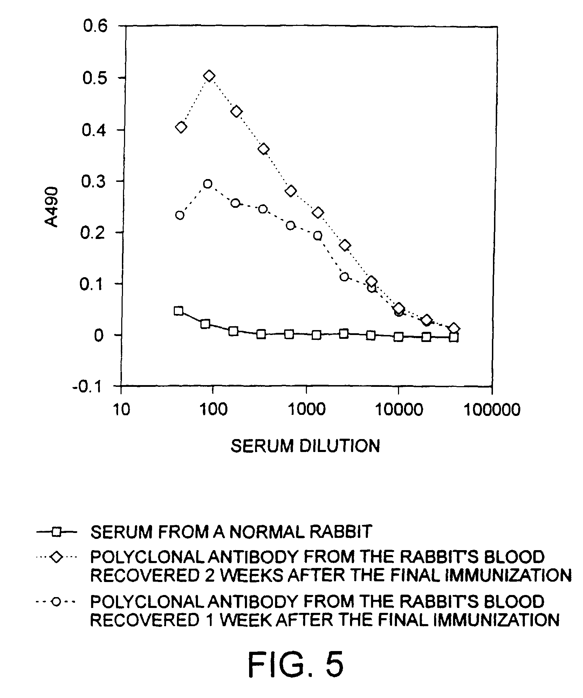

These results are shown in Fig. 5. In Fig. 5, the axis of abscissas represents concentrations of the added polyclonal antibody or the number of dilutions of the serum, and the axis of ordinates represents the absorbance at 490 nm. In this figure, the antibody titers derived from an immunized rabbit are greater than the control. Thus the polyclonal antibody against the peptide of sequence 1 was obtained.

### (EXAMPLE 4) Western Blotting

The P-dlg antibody obtained in Example 3 was used to conduct western blotting. The protocol is shown below.
(I) COS cells into which the P-dlg gene was introduced and the cells of their parents' strain (cells having no P-dlg gene: designated "Mock") were provided. 1x10⁶ cells were dissolved as described below and its supernatant was recovered.
   The composition of a cell lysate comprises 50 mM Tris-HCl, 150 mM NaCl, 1% Triton X-100, 50 mM iodoacetamide, 2 mM MgCl₂, 2 mM CaCl₂, 0.1% NaN₃, 10 µg/ml soybean trypsin inhibitor, 1 µg/ml aprotinin, 1 mM PMSF (phenyl methyl sulfonyl fluoride), and 1 µg/ml leupeptin. This cell lysing solution (100 µl) was added to the cell pellet, and dissolved by stirring. After standing on ice for 60 min, the solution was centrifuged at 15,000 rpm for 10 min and the supernatant was recovered.
(II) To the recovered supernatant was added 50 µl of the CNBr-activated Sepharose beads (available from Amersham Pharmacia Biotech Inc.; antibody concentration of 1 mg/1 ml resin) to which 1 mg of IgG from a non-immunized rabbit had been bound. After overnight reaction at 4 °C, it was centrifuged to remove nonspecific binding proteins bound to the beads and the supernatant was recovered.
(III) 10 microliters of the supernatant obtained in item (II) was mixed with the same quantity of the sample buffer for SDS-PAGE and 1 µl of 2-mercaptoethanol. After heat treatment at 95 °C for 5 min, it was electrophoresed on a 5-20% gradient gel.
(IV) After electrophoresis, the migrated proteins were transferred onto a nitrocellulose membrane using a Trans Blot System (available from Marisol Inc.).
(V) The nitrocellulose membrane was immersed in Blockace (available from Dainippon Pharmaceutical Co. Ltd.) and left for 1 h to effect blocking. Subsequently, it was twice washed with 0.05% Tween20/PBS for 5 min.
(VI) The anti-P-dlg antibody against sequence 1 as prepared in Example 3 was diluted with PBS to give a concentration of 1 µg/ml, and it was added to the membrane and allowed to react at room temperature for 2 h. Subsequently, the membrane was three times washed with 0.05% Tween20/PBS for 5 min.
(VII) A peroxidase-labeled anti rabbit IgG solution (available from Caltag Inc.) was diluted with PBS 1000-fold and the solution was added to the above-described membrane and further allowed to react at room temperature for 1 h. Subsequently, the membrane was three times washed with 0.05% Tween20/PBS for 5 min.
(VIII) Five milliliters of the coloring solution from an ECL kit (available from Amasham Pharmacia Biotech Inc.) was added to the membrane and it was allowed to react for 1 min. Then, this membrane was exposed to an X-ray film for 20 sec and the film developed, then, a photograph was taken. The result is shown in Fig. 6. In Fig. 6, the band for the P-dlg molecule was recognized at the position of about 110 kd in the COS cells. This molecular weight is almost in agreement with that expected based on the amino acid sequence of P-dlg. However, this band was not observed in Mock.

### (EXAMPLE 5) Tissue Staining

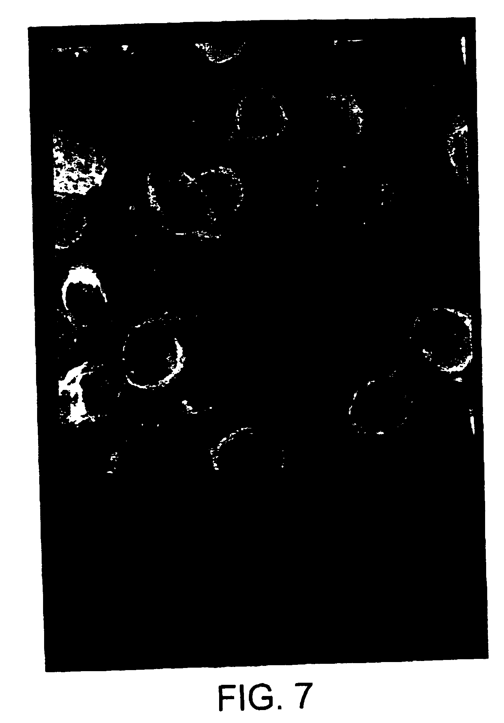

Employing the antibody obtained in Example 3, the P-dlg expression state in the cell in which P-dlg was expressed was examined by immunostaining of tissues. Human prostate cell strain PC-3 was used as the material and the staining was carried out as described below.
(I) Cell was cultured in Labtek chamber slide.
(II) Next, slide cultured cell was treated with 3.7% formaldehyde for 3 min, and then treated with methanol chilled at -20 °C for 3 min to effect fixing. Subsequently, the slide was washed with 50 mM Tris-HCl (pH 7.5) (5 min three times).
(III) Blockace (available from Dainippon Pharmaceutical Co. Ltd.) was placed over the tissue on the slide glass and blocking was conducted by allowing them to stand at room temperature for 1 h. Subsequently, the slide was washed with 50 mM Tris-HCl (pH 7.5) (5 min three times).
(IV) The anti-P-dlg antibody against sequence 1 as prepared in Example 3 was diluted with PBS to give a concentration of 1 µg/ml, and it was added onto the cell on the slide and allowed to react at room temperature for 1 h. Subsequently, the slide was washed with 50 mM Tris-HCl (pH 7.5) (5 min three times).
(V) A FITC-labeled anti rabbit IgG preparation (available from Caltag Inc.) diluted with PBS 1000-fold was added onto the cell on the slide and allowed to react at room temperature for 1 h. Subsequently, the slide was washed with 50 mM Iris-HCl (pH 7.5) (5 min three times).
(VI) The slide thus obtained was observed under a fluorescence microscope. The results are shown in Fig. 7. It was ascertained from Fig. 7 that P-dlg was intensively expressed in boundary portion between cytoplasm and cell membrane.

### INDUSTRIAL APPLICABILITY

The present invention provides novel dlg family molecules derived from human being, antibodies against the same and genes encoding the molecules. Further, the present invention provides polynucleotide probes for detecting the polynucleotides in specimens.

Furthermore, P-dlg gene was found to be expressed inventively in prostate. In prostatic cancer, a deletion of long-arm of the chromosome 10, where P-dlg gene of the present invention is located, is frequently observed, and therefore the P-dlg gene of the present invention can provide useful information in respect to prostatic cancer. Moreover, the P-dlg of the present invention can also provide the useful information for clarifying the mechanism of carcinogenesis of prostate. In addition, the P-dlg of the present invention is useful in clarifying the mechanism of function of the dlg family molecules as intracellular protein, and the mechanism of intracellular protein transportation.

Sequence Listing

SEQ ID NO: 1

Sequence Length: 674

Sequence Type: amino acid

Topology: straight chain

Kind of Sequence: peptide

Sequence:

Met Arg Ala Thr His Gly Ser Asn Ser Leu Pro Ser Ser Ala Arg Leu

1               5                   10                  15

Gly Ser Ser Ser Asn Leu Gln Phe Lys Ala Glu Arg Ile Lys Ile Pro

            20                  25                  30

Ser Thr Pro Arg Tyr Pro Arg Ser Val Val Gly Ser Glu Arg Gly Ser

        35                  40                  45

Val Ser His Ser Glu Cys Ser Thr Pro Pro Gln Ser Pro Leu Asn Ile

    50                  55                  60

Asp Thr Leu Ser Ser Cys Ser Gln Ser Gln Thr Ser Ala Ser Thr Leu

65                  70                  75                  80

Pro Arg Ile Ala Val Asn Pro Ala Ser Leu Gly Glu Arg Arg Lys Asp

                85                  90                  95

Arg Pro Tyr Val Glu Glu Pro Arg His Val Lys Val Gln Lys Gly Ser

            100                 105                 110

Glu Pro Leu Gly Ile Ser Ile Val Ser Gly Glu Lys Gly Gly Ile Tyr

        115                 120                 125

Val Ser Lys Val Thr Val Gly Ser Ile Ala His Gln Ala Gly Leu Glu

    130                 135                 140

Tyr Gly Asp Gln Leu Leu Glu Phe Asn Gly Ile Asn Leu Arg Ser Ala

145                 150                 155                 160

Thr Glu Gln Gln Ala Arg Leu Ile Ile Gly Gln Gln Cys Asp Thr Ile

                165                 170                 175

Thr Ile Leu Ala Gln Tyr Asn Pro His Val His Gln Leu Ser Ser His

            180                 185                 190

Ser Arg Ser Ser Ser His Leu Asp Pro Ala Gly Thr His Ser Thr Leu

        195                 200                 205

Gln Gly Ser Gly Thr Thr Thr Pro Glu His Pro Ser Val Ile Asp Pro

    210                 215                 220

Leu Met Glu Gln Asp Glu Gly Pro Ser Thr Pro Pro Ala Lys Gln Ser

225                 230                 235                 240

Ser Ser Arg Ile Ala Gly Asp Ala Asn Lys Lys Thr Leu Glu Pro Arg

                245                 250                 255

Val Val Phe Ile Lys Lys Ser Gln Leu Glu Leu Gly Val His Leu Cys

            260                 265                 270

Gly Gly Asn Leu His Gly Val Phe Val Ala Glu Val Glu Asp Asp Ser

        275                 280                 285

Pro Ala Lys Gly Pro Asp Gly Leu Val Pro Gly Asp Leu Ile Leu Glu

    290                 295                 300

Tyr Gly Ser Leu Asp Val Arg Asn Lys Thr Val Glu Glu Val Tyr Val

305                 310                 315                 320

Glu Met Leu Lys Pro Arg Asp Gly Val Arg Leu Lys Val Gln Tyr Arg

                325                 330                 335

Pro Glu Glu Phe Thr Lys Ala Lys Gly Leu Pro Gly Asp Ser Phe Tyr

            340                 345                 350

Ile Arg Ala Leu Tyr Asp Arg Leu Ala Asp Val Glu Gln Glu Leu Ser

        355                 360                 365
Phe Lys Lys Asp Asp Ile Leu Tyr Val Asp Asp Thr Leu Pro Gln Gly
    370                 375                 380
Thr Phe Gly Ser Trp Met Ala Trp Gln Leu Asp Glu Asn Ala Gln Lys
385                 390                 395                 400
Ile Gln Arg Gly Gln Ile Pro Ser Lys Tyr Val Met Asp Gln Glu Phe
                405                 410                 415
Ser Arg Arg Leu Ser Met Ser Glu Val Lys Asp Asp Asn Ser Ala Thr
            420                 425                 430
Lys Thr Leu Ser Ala Ala Ala Arg Arg Ser Phe Phe Arg Arg Lys His
        435                 440                 445
Lys His Lys Arg Ser Gly Ser Lys Asp Gly Lys Asp Leu Leu Ala Leu
    450                 455                 460
Asp Ala Phe Ser Ser Asp Ser Ile Pro Leu Phe Glu Asp Ser Val Ser
465                 470                 475                 480
Leu Ala Tyr Gln Arg Val Gln Lys Val Asp Cys Thr Ala Leu Arg Pro
                485                 490                 495
Val Leu Ile Leu Gly Pro Leu Leu Asp Val Val Lys Glu Met Leu Val
            500                 505                 510
Asn Glu Ala Pro Gly Lys Phe Cys Arg Cys Pro Leu Glu Val Met Lys
        515                 520                 525
Ala Ser Gln Gln Ala Ile Glu Arg Gly Val Lys Asp Cys Leu Phe Val
    530                 535                 540
Asp Tyr Lys Arg Arg Ser Gly His Phe Asp Val Thr Thr Val Ala Ser
545                 550                 555                 560
Ile Xaa Glu Ile Thr Glu Lys Asn Arg His Cys Leu Leu Asp Ile Ala

                565                 570                 575
Pro His Ala Ile Glu Arg Leu His His Met His Ile Tyr Pro Ile Val
            580                 585                 590
Ile Phe Ile His Tyr Lys Ser Ala Lys His Ile Lys Glu Gln Arg Asp
        595                 600                 605
Pro Ile Tyr Leu Arg Asp Lys Val Thr Gln Arg His Ser Lys Glu Gln
    610                 615                 620
Phe Glu Ala Ala Gln Lys Leu Glu Gln Glu Tyr Ser Arg Tyr Phe Thr
625                 630                 635                 640
Gly Val Ile Gln Gly Gly Ala Leu Ser Ser Ile Cys Thr Gln Ile Leu
                645                 650                 655
Ala Met Val Asn Gln Glu Gln Asn Lys Val Leu Trp Ile Pro Ala Cys
            660                 665                 670
Pro Leu

SEQ ID NO: 2
Sequence Length: 3035
Sequence Type: nucleic acie
Topology: straight chain
Kind of Sequence: cDNA to mRNA
Sequence:
TAGCAGACAC TCTTGCCCTT GCATCTGCAG GAACAGAAGT GTGTCCCGGC CAGTGGAGAA 60
CTCTCCCCGG AGCTCCAGGA GTGGGCACCT TACTCGCCTG GGCATTCCAG CCGGCACAGC 120
AACCCCCCGC TATACCCTAG CAGGCCGTCT GTGGGCACTG TTCCCCGGAG TTTGACCCCC 180
AGCACCACTG TGAGCTCCAT CCTGCGGAAC CCCATCTACA CTGTGCGCAG TCACAGGGTC 240
GGCCCCTGCA GCTCTCCACC TGCGGCCCGA GATGCTGGCC CCCAGGGTTT GCATCCCAGT 300

GTCCAGCACC AGGGACGCCT GAGCCTGGAC CTGAGCCACA GGACCTGCAG CGACTACTCC 360
GAG ATG AGA GCC ACC CAT GGG TCC AAC TCA CTG CCC TCC AGC GCC CGC 408
CTG GGT TCT TCG AGT AAC TTG CAG TTC AAG GCG GAA CGC ATT AAA ATC 456
CCA TCA ACA CCA AGA TAT CCG CGG AGT GTC GTG GGC TCC GAG AGA GGT 504
TCA GTG TCA CAT TCT GAA TGC AGC ACT CCT CCA CAG TCA CCC CTG AAC 552
ATC GAC ACC CTG TCC TCT TGT AGC CAG TCC CAG ACC TCA GCC TCC ACA 600
TTG CCC AGA ATC GCT GTC AAC CCC GCG TCC CTC GGG GAG CGG AGA AAG 648
GAC AGG CCT TAT GTG GAG GAG CCA CGC CAC GTG AAG GTG CAG AAG GGC 696
TCA GAG CCG CTG GGC ATC TCC ATC GTG AGT GGA GAG AAG GGC GGC ATC 744
TAC GTC TCC AAG GTG ACC GTG GGG AGC ATC GCT CAC CAG GCT GGC CTC 792
GAG TAT GGG GAT CAG TTA CTG GAG TTC AAC GGC ATA AAC CTG CGG AGC 840
GCC ACG GAG CAG CAG GCG CGG CTC ATC ATC GGG CAG CAG TGT GAT ACC 888
ATC ACC ATC CTG GCC CAG TAC AAC CCC CAC GTG CAC CAG CTC AGC AGC 936
CAC TCC CGG TCC AGC TCA CAC CTG GAC CCT GCC GGT ACC CAC TCC ACT 984
CTC CAG GGC AGT GGC ACC ACC ACC CCG GAG CAT CCA TCT GTC ATC GAC 1032
CCA CTG ATG GAG CAG GAC GAG GGG CCT AGC ACC CCC CCA GCC AAG CAG 1080
AGC AGC TCC AGG ATT GCG GGA GAT GCC AAC AAG AAG ACC CTG GAG CCA 1128
CGC GTT GTC TTC ATC AAA AAG TCC CAG CTG GAG CTT GGG GTG CAC TTG 1176
TGT GGT GGG AAC CTG CAT GGG GTG TTT GTG GCC GAG GTG GAG GAT GAC 1224
AGT CCT GCC AAG GGT CCT GAC GGC CTC GTG CCA GGG GAC CTC ATC CTG 1272
GAG TAT GGC AGC CTG GAC GTG CGG AAC AAG ACA GTG GAG GAA GTC TAT 1320
GTG GAG ATG CTG AAG CCC AGG GAT GGC GTC CGC CTG AAG GTG CAG TAC 1368
CGC CCT GAG GAG TTC ACG AAG GCC AAG GGC CTG CCT GGT GAC AGC TTC 1416
TAC ATC AGG GCC CTG TAC GAC CGG CTG GCA GAT GTG GAG CAA GAG TTG 1464
AGC TTT AAG AAG GAC GAC ATC CTC TAC GTG GAT GAC ACC TTA CCC CAG 1512
GGC ACG TTC GGG TCC TGG ATG GCT TGG CAG CTG GAC GAG AAT GCC CAG 1560

AAG ATC CAG CGC GGG CAG ATT CCC AGC AAA TAT GTG ATG GAC CAA GAA 1608
TTC TCC AGG AGG CTC AGC ATG TCT GAA GTC AAA GAT GAC AAT AGC GCC 1656
ACA AAG ACG CTG TCA GCG GCT GCA CGC CGG TCC TTT TTT CGG AGG AAA 1704
CAC AAG CAC AAA CGC AGC GGG TCC AAG GAC GGG AAA GAC CTG CTC GCC 1752
TTG GAT GCC TTT TCC AGT GAC TCC ATT CCA CTC TTT GAA GAT TCG GTG 1800
AGC CTG GCC TAT CAG CGG GTC CAG AAG GTG GAC TGC ACC GCT CTG AGG 1848
CCT GTC CTG ATT CTG GGG CCT TTG CTG GAC GTG GTG AAG GAG ATG CTG 1896
GTG AAT GAG GCT CCT GGC AAG TTC TGC AGA TGT CCC CTT GAG GTG ATG 1944
AAG GCC TCC CAG CAG GCC ATT GAG CGG GGT GTC AAA GAT TGC CTG TTT 1992
GTC GAC TAT AAG CGG AGA AGC GGC CAT TTC GAT GTG ACC ACT GTG GCG 2040
TCA ATA WAG GAG ATC ACA GAA AAG AAC CGA CAC TGC CTC CTG GAC ATT 2088
GCT CCG CAC GCT ATT GAG CGG CTC CAC CAC ATG CAC ATC TAC CCC ATT 2136
GTC ATC TTC ATC CAC TAC AAG AGC GCC AAG CAC ATC AAG GAG CAG AGA 2184
GAC CCC ATC TAC CTG AGG GAC AAG GTG ACT CAG AGG CAT TCC AAA GAG 2232
CAR TTT GAG GCG GCG CAG AAG CTT GAG CAG GAG TAC AGC AGG TAC TTC 2280
ACA GGG GTC ATC CAG GGA GGA GCC CTG TCA AGC ATT TGC ACT CAG ATC 2328
TTG GCA ATG GTC AAT CAA GAA CAA AAT AAA GTC CTG TGG ATT CCA GCC 2376
TGC CCG CTC TAG GAGAATGCTG TGCTGTGGAT GACTGCAGCT GGCCGCCTGA 2428
GGGGACACCA GACTCAGCTC TTTTCTAGCG ACTGAAAGTA GAAGTCTGTC CGTCTATGAA 2488
CATGCGGGGG AAGGATCCGG AACCAGGACC CAGAAGCACC TCCTTTGTAG ACAGAGGGCC 2548
ACGGCTGCGT GCGATCCAGG CCCAGGMCCA CACACTCTGC CCGTGTCACA CGTGTGCTTT 2608
AACACAAAAC AGATAACACT AAAGACGGGT TCAGCACCCA CCTTTCTTTA GCCAGCTGAT 2668
CAGAGATGCT GCAAAGAGAA CCTTTCGGAT CACTCGTTTA CAAGCCTTTT CTAAGTATTT 2728
GGTGGTTTAT GTTTACTTGA ACGGCTCCAT GTTGCCGGTG CCCAGCCCCT GTCCCCTCTG 2788
TCAACCCCCT GTCGCTTTGG TGTTGGTTTC GTTCCCGTCT TCAGCAAAAC GACCTTGGAA 2848
CCTCAATGGG GGCTGCTTTG CTTTGGGAGG TTCTTGTTGG TGGGACCAGA GCTTTGACAA 2908

ACCTCCTGCT CCTTGGTGGG CACCTCTCCT GGGAAGGACG TTCACAACTC CAGGGTGCTT 2968
CAGAATGCCT GTGGAACMAG GAACCAGTGG CCTTTGGATT TTTTCCTCCC ACAATGGGGG 3028
AAGGTGA 3035

SEQ ID NO: 3
Sequence Length: 1504
Sequence Type: nucleic acid
Topology: straight chain
Kind of Sequence: cDNA to mRNA
Sequence:
TCCAGGAGGC TCAGCATGTC TGAAGTCAAA GATGACAATA GCGCCACAAA GACGCTGTCA 60
GCGGCTGCAC GCCGGTSCTT TTTTCGGAGG AAACACAAGC ACAAACGCAG CGGGTCCAAG 120
GACGGGAAAG ACCTGCTCGC CTTGGATGCC TTTTCCAGTG ACTCCATTCC ACTCTTTGAA 180
GATTCGGTGA GCCTGGCCTA TCAGCGGGTC CAGAAGGTGG ACTGCACCGC TCTGAGGCCT 240
GTCCTGATTC TGGGGCCTTT GCTGGACGTG GTGAAGGAGA TGCTGGTGAA TGAGGCTCCT 300
GGCAAGTTCT GCAGATGTCC CCTTGAGGTG ATGAAGGCCT CCCAGCAGGC CATTGAGCGG 360
GGTGTCAAAG ATTGCCTGTT TGTCGACTAT AAGCGGAGAA GCGGCCATTT CGATGTGACC 420
ACTGTGGCGT CAATAWAGGA GATCACAGAA AAGAACCGAC ACTGCCTCCT GGACATTGCT 480
CCGCACGCTA TTGAGCGGCT CCACCACATG CACATCTACC CCATTGTCAT CTTCATCCAC 600
TACAAGAGCG CCAAGCACAT CAAGGAGCAG AGAGACCCCA TCTACCTGAG GGACAAGGTG 660
ACTCAGAGGC ATTCCAAAGA GCARTTTGAG GCGGCGCAGA AGCTTGAGCA GGAGTACAGC 720
AGGTACTTCA CAGGGGTCAT CCAGGGAGGA GCCCTGTCAA GCATTTGCAC TCAGATCTTG 780
GCAATGGTCA ATCAAGAACA AAATAAAGTC CTGTGGATTC CAGCCTGCCC GCTCTAGGAG 860
AATGCTGTGC TGTGGATGAC TGCAGCTGGC CGCCTGAGGG GACACCAGAC TCAGCTCTTT 920
TCTAGCGACT GAAAGTAGAA GTCTGTCCGT CTATGAACAT GCGGGGGAAG GATCCGGAAC 980
CAGGACCCAG AAGCACCTCC TTTGTAGACA GAGGGCCACG GCTGCGTGCG ATCCAGGCCC 1040

AGGMCCACAC ACTCTGCCCG TGTCACACGT GTGCTTTAAC ACAAAACAGA TAACACTAAA 1100

GACGGGTTCA GCACCCACCT TTCTTTAGCC AGCTGATCAG AGATGCTGCA AAGAGAACCT 1160

TTCGGATCAC TCGTTTACAA GCCTTTTCTA AGTATTTGGT GGTTTATGTT TACTTGAACG 1220

GCTCCATGTT GCCGGTGCCC AGCCCCTGTC CCCTCTGTCA ACCCCCTGTC GCTTTGGTGT 1280

TGGTTTCGTT CCCGTCTTCA GCAAAACGAC CTTGGAACCT CAATGGGGGC TGCTTTGCTT 1340

TGGGAGGTTC TTGTTGGTGG GACCAGAGCT TTGACAAACC TCCTGCTCCT TGGTGGGCAC 1400

CTCTCCTGGG AAGGACGTTC ACAACTCCAG GGTGCTTCAG AATGCCTGTG GAACMAGGAA 1460

CCAGTGGCCT TTGGATTTTT TCCTCCCACA ATGGGGGAAG GTGA 1504

SEQ ID NO: 4

Sequence Length: 23

Sequence Type: nucleic acid

Topology: straight chain

Kind of Sequence: another nucleic acid

Sequence:

GCATCCAAGG CGAGCAGGTC TTT 23

SEQ ID NO: 5

Sequence Length: 24

Sequence Type: nucleic acid

Topology: straight chain

Kind of Sequence: another nucleic acid

Sequence:

TGCAGCCGCT GACAGCGTCT TTGT 24

SEQ ID NO: 6

Sequence Length: 17
Sequence Type: nucleic acid
Topology: straight chain
Kind of Sequence: another nucleic acid
Sequence:
AATACGACTC ACTATAG 17

SEQ ID NO: 7
Sequence Length: 24
Sequence Type: nucleic acid
Topology: straight chain
Kind of Sequence: another nucleic acid
Sequence:
AAAGACAACC CCAGGATTCG GAAG 24

SEQ ID NO: 8
Sequence Length: 24
Sequence Type: nucleic acid
Topology: straight chain
Kind of Sequence: another nucleic acid
Sequence:
CGTGAACTCC TCAGGGCGGT ACTG 24

SEQ ID NO: 9
Sequence Length: 21
Sequence Type: nucleic acid

Topology: straight chain
Kind of Sequence: another nucleic acid
Sequence:
CTTCAGGCGG ACGCCAGCCC T 21

SEQ ID NO: 10
Sequence Length: 17
Sequence Type: nucleic acid
Topology: straight chain
Kind of Sequence: another nucleic acid
Sequence:
ATTAACCCTC ACTAAAG 17

SEQ ID NO: 11
Sequence Length: 23
Sequence Type: nucleic acid
Topology: straight chain
Kind of Sequence: another nucleic acid
Sequence:
TAGCAGACAC TCTTGCCCTT GCA 23

SEQ ID NO: 12
Sequence Length: 24
Sequence Type: nucleic acid
Topology: straight chain
Kind of Sequence: another nucleic acid

Sequence:

TCACCTTCCC CCATTGTGGG AGGA 24

## Claims

1. A dlg family molecule having an amino acid sequence set forth in SEQ ID NO: 1 of the Sequence Listing.

2. A dlg family molecule according to claim 1 wherein one or more amino acids of the amino acid sequence set forth in SEQ ID NO:1 of the Sequence Listing are substituted, deleted or added, said dlg family molecule having a function of reacting with an antibody against Lys-Glu-Gln-Arg-Asp-Pro-Ile-Tyr-Leu-Arg-Asp-Lys-Val-Thr-Gln-Arg-His-Ser-Lys-Glu.

3. A polynucleotide encoding the dlg family molecule according to claim 1 or 2.

4. An antibody against the dlg family molecule according to claim 1 or 2.

5. A polynucleotide having a base sequence comprising 12 or more consecutive bases in the base sequence set forth in SEQ ID NO:2 of the Sequence Listing, said the polynucleotide being a portion of the polynucleotide according to claim 3.

6. A polynucleotide containing an antisense base sequence corresponding to the polynucleotide according to claim 5.

7. A derivative of the polynucleotide according to claim 5 or 6.

8. A polynucleotide having a base sequence comprising 364th to 2388th bases in SEQ ID NO:2 in the Sequence Listing.

9. An antibody according to claim 4 against an epitope having an amino acid sequence consisting of KEQRDPIYLRDKVTQRHSKE.
